# EUROPÄISCHE PATENTANMELDUNG

(11) **EP 2 529 778 A2**
(43) Veröffentlichungstag der Anmeldung: **05.12.2012**
(21) Anmeldenummer: 12181913.0
(22) Anmeldetag: 19.12.2008
(51) Int. Cl.: A61M 11/06, A61M 11/00

(54) **Vorrichtung zum Zerstäuben von Fluiden**

(30) Priorität: 08.01.2008 DE 102008003542
(62) Teilanmeldung aus: 08869999.6
(71) Anmelder: Medic Activ Vertriebs GmbH, 82031 Grünwald (DE); Brunner Maschinenbau GmbH, 85737 Ismaning (DE)
(72) Erfinder: Brunner, Peter, 85737 Ismaning (DE); Lindena, Bernd-Heiko, 6363 Westendorf (AT)
(74) Vertreter: Dilg, Haeusler, Schindelmann Patentanwaltsgesellschaft mbH

(57) **Zusammenfassung**

Vorrichtung zum Zerstäuben wenigstens eines Fluids, insbesondere zur Erzeugung eines therapeutisch wirksamen Aerosols mit wenigstens einem Aerosolerzeuger (10, 60) zur Abgabe eines Aerosols, dadurch gekennzeichnet, dass der Aerosolerzeuger (10, 60) ein zu zerstäubendes Fluid mit Druck beaufschlagt und mit einer Fließgeschwindigkeit im Bereich von 50 bis 300 m/s durch wenigstens eine Austrittsöffnung (14, 17) in Form von kleinen Partikeln ausstößt.

## Beschreibung

Die vorliegende Erfindung betrifft ein Verfahren und eine Vorrichtung zum Zerstäuben von Fluiden, insbesondere ein Verfahren und eine Vorrichtung zur Erzeugung eines therapeutisch wirksamen Aerosols in einem Behandlungsraum, beispielsweise insbesondere zur Behandlung von Menschen oder Tieren mit Aerosolen, die durch Zerstäuben von wässrigen und/oder öligen Lösungen erzeugt werden.

Seit langem ist die heilsame Wirkung von Dämpfen oder Aerosolnebeln mit fein verteilten Wasser- oder Öltröpfchen bekannt. In herkömmlichen Sauna- oder Dampfbädern wird dazu Wasser oder eine geringe Menge ätherische Öle enthaltende wässrige Emulsion verdampft. Der heiße Dampf kühlt im Saunaraum ab und kondensiert zu Nebeltröpfchen. Ein Nachteil der Nebelerzeugung durch Verdampfen von Wasser oder einer wässrigen Ölemulsion liegt in der hohen Temperatur des erzeugten Nebels. Während die anregende Wirkung des heißen Nebels für bestimmte Anwendungen durchaus förderlich sein kann, sind herkömmliche Sauna- und Dampfbäder jedoch stets mit dem Nachteil verbunden, dass der Kreislauf der badenden Person hohen Belastungen ausgesetzt ist und daher Sauna- und Dampfbäder für große Bevölkerungsgruppen kontraindiziert sind.

Es ist daher bereits vorgeschlagen worden, wässrige Lösungen oder Emulsionen bei niedrigeren Temperaturen, typischerweise bei Temperaturen von weniger als 35 oder 40 °C, bevorzugt in einem Temperaturbereich von 22 bis 28 °C, zu zerstäuben. Damit wird der Kreislauf der zu behandelnden Person im Vergleich zu herkömmlichen Sauna- oder Dampfbädern weniger stark belastet.

In der internationalen Patentanmeldung WO 00/44331 A1 wird eine Ganzkörper-Nebelbadvorrichtung und ein Verfahren zum Verabreichen eines Ganzkörper-Nebelbades beschrieben, wo bei Temperaturen von weniger als 35 °C ein Nebelbad erzeugt wird, wobei man eine Flüssigkeit auf einen Druck von mehr als 100 bar komprimiert und anschließend über eine Düse explosionsartig in eine Behandlungskabine ausstößt, so dass die Flüssigkeit beim Ausstoßen in die Kabine in Folge ihres hohen inneren Drucks in viele kleine Flüssigkeitströpfchen zerstäubt wird. Bereits in WO 00/44331 A1 ist vorgeschlagen worden, wässrige Salzlösungen oder Öle mit Vitaminzusätzen oder ätherischen Zusätzen zur Erzeugung des Nebels zu verwenden. Wenn ein Nebel aus zwei unterschiedlichen Flüssigkeiten erzeugt werden soll, werden gemäß WO 00/44331 zwei separate Vernebler verwendet. Bei den in diesem Dokument beschriebenen Verneblern ist ein hohlzylinderförmiger Kompressionsraum mit einer verschließbaren Öffnung vorgesehen, der über eine Leitung mit einer Hochdruckpumpe verbunden ist, aus der die zu vernebelnde Flüssigkeit gefördert wird.

Nachteilig an der aus diesem Stand der Technik bekannten Lösung ist einerseits, dass für jedes zu zerstäubende Fluid ein separater Vernebler mit separater Hochdruckpumpe erforderlich ist. Außerdem stellt die Verwendung einer Hochdruckpumpe in einer feuchten Umgebung ein Sicherheitsrisiko dar, insbesondere wenn wässrige Salzlösungen vernebelt werden, die eine wesentlich höhere Leitfähigkeit als normales Leitungswasser aufweisen.

Der vorliegenden Erfindung liegt daher das technische Problem zu Grunde, ein Verfahren und eine Vorrichtung zum Zerstäuben von wenigsten zwei unterschiedlichen Fluiden, insbesondere zur Behandlung von Menschen oder Tieren mit wässrigen und öligen Aerosolen, bereitzustellen, die einfach aufgebaut ist und die zuverlässig und sicher in einer feuchten Umgebung, in der sich beispielsweise Aerosole aus wässrigen Salzlösungen befinden, betreibbar ist.

Gelöst wird dieses technische Problem durch das Verfahren des vorliegenden Patentanspruchs 1 und die Vorrichtung des vorliegenden Patentanspruchs 13. Vorteilhafte Weiterbildungen der erfindungsgemäßen Vorrichtung sind Gegenstände der abhängigen Patentansprüche.

Die Erfindung betrifft demnach ein Verfahren zur Erzeugung eines therapeutisch wirksamen Aerosols in einem Behandlungsraum, wobei man ein zu zerstäubendes Fluid mit einem Druck beaufschlagt und mit einer Fließgeschwindigkeit im Bereich von 50 bis 3000 m/s durch eine Düse in einen Behandlungsraum ausstößt. Die Fließgeschwindigkeit bezieht sich dabei auf die Fließgeschwindigkeit des Fluids an der Düse. Die Düse hat vorzugsweise wenigstens eine Austrittsöffnung mit einen Durchmesser auf, der im Bereich von 0,05 bis 0,2 mm, bevorzugt im Bereich von 0,08 bis 0,15 mm und ganz besonders bevorzugt etwa im Bereich von 0,1 mm liegt. Beim Durchströmen der feinen Düse wird das Fluid in kleinste Partikel zerrissen, die im Behandlungsraum ein Aerosol bilden. Die Austrittsöffnung der Düse kann aus jeglichem abriebfesten und gegenüber den zu zerstäubenden Fluiden beständigen Material bestehen, beispielsweise aus einem geeigneten Kunststoff- oder Keramikmaterial. Vorteilhaft besteht die Düse aus einem rost- und säurebeständigem Edelstahl und kann beispielsweise als Platte mit einer Dicke von 0,5 bis 1 mm ausgebildet sein. Die Düse kann eine oder mehrere Austrittsöffnung aufweisen. Die Austrittsöffnungen können beispielsweise mechanisch oder mit mittels Laserstrahl oder Hochdruckwasserstrahl gebohrt werden. Alternativ ist es auch möglich, die Düse als Spritzgussteil herzustellen, wobei die entsprechenden Düsenöffnungen bereits beim Herstellungsprozess ausgespart bleiben.

Die Fließgeschwindigkeit des Fluids beim Verlassen der Düse liegt bevorzugt im Bereich von 100 bis 250 m/s und besonders bevorzugt bei etwa 150 m/s.

Damit das Fluid die gewünschte Fließgeschwindigkeit erreicht, wird das zu zerstäubende Fluid vorzugsweise mit einem Druck beaufschlagt, der bis zu 1.000 bar betragen kann.

Die ausgestoßenen Partikel weisen einen mittleren Durchmesser auf, der auf den jeweiligen Anwendungsbereich des Aerosols abgestimmt ist. Vorzugsweise liegt der mittlere Partikeldurchmesser bei 0,05 bis 5 µm, besonders bevorzugt im Bereich von 0,1 bis 0,9 µm, da Partikel in diesem Größenordnungsbereich eine gute Lungengängigkeit aufweisen, so dass eine besonders effektive Resorption gewährleistet ist. Bei topischen Anwendungen können die Partikeldurchmesser auch wesentlich größer sein, da in diesem Fall lediglich gewährleistet sein muss, dass die Tröpfchen des zerstäubten Fluids die Haut erreichen.

Gemäß einer besonders bevorzugten Ausführungsform ist das zu zerstäubende Fluid eine wässrige Lösung, eine wässrige Emulsion, insbesondere eine Öl-in-Wasser-Emulsion, eine ölige Lösung, eine ölige Emulsion, insbesondere eine Wasser-in-ÖlEmulsion, eine wässrige Suspension oder eine ölige Suspension. Dabei handelt es sich bei der wässrigen Lösung besonders bevorzugt um eine Salzlösung. Im Fall einer öligen Lösung handelt es sich bevorzugt um eine Lösung, die ätherische Öle umfasst.

Je nach Anwendungsfall kann das Fluid wenigstens einen therapeutisch wirksamen Bestandteil umfassen, beispielsweise bei topischer Anwendung Medikamente zur Behandlung von Hauterkrankungen oder bei Inhalation des Aerosols Medikamente zur Behandlung von Atemwegserkrankungen.

Als Behandlungsraum kann jeglicher Raum dienen, der abgestimmt auf den jeweiligen Anwendungsfall ermöglicht, dass die Haut oder die Atemwege des zu behandelnden Menschen oder des zu behandelnden Tieres mit den zerstäubten Aerosolpartikeln in Kontakt kommen. Daher kann der Behandlungsraum beispielsweise auch eine Inhalationsmaske sein. Vorzugsweise ist der Behandlungsraum jedoch eine Behandlungskammer, welche das mit dem Aerosol zu behandelnde Lebewesen ganz oder teilweise umgibt.

Eine Besonderheit des erfindungsgemäßen Verfahrens liegt insbesondere im Vergleich zum typischen Dampfbad darin, dass die Behandlung bei relativ niedrigen Temperaturen durchgeführt werden kann, da keine thermische Verdampfung von Fluiden zur Aerosolerzeugung notwendig ist. Die bevorzugte Temperatur in der Behandlungskammer liegt bei Umgebungstemperatur oder leicht über Umgebungstemperatur. In besonders heißen Regionen der Erde kann aber auch eine Kühlung der Behandlungskammer auf Temperaturen unterhalb der Umgebungstemperatur vorteilhaft sein. Bevorzugt ist die Behandlungskammer daher auf eine Temperatur im Bereich von 0 bis 70 °C, bevorzugt im Bereich von 20 bis 35 °C temperierbar.

Die typische Behandlungsdauer mit dem Aerosol liegt im Bereich von Minuten, beispielsweise im Bereich von 10 bis 30 Minuten. Dabei wird das Aerosol je nach Volumen des Behandlungsraums mit einer durchschnittlichen Durchflussphase im Bereich von wenigen Millilitern pro Minute bis zu einigen hundert Millilitern pro Minute ausgestoßen. Besonders bevorzugt liegt die durchschnittliche Durchflussrate im Bereich von 10 bis 100 Millilitern pro Minute.

Die Erfindung betrifft außerdem eine Vorrichtung zum Zerstäuben von Fluiden, insbesondere eine Vorrichtung zur Erzeugung eines therapeutisch wirksamen Aerosols, mit einem Behandlungsraum, welcher ein zu behandelndes Lebewesen ganz oder teilweise umgibt, und wenigstens einem Aerosolerzeuger zur Abgabe eines Aerosols in den Behandlungsraum, wobei der Aerosolerzeuger so ausgebildet ist, dass ein zu zerstäubendes Fluid mit einem Druck beaufschlagt und mit einer Fließgeschwindigkeit im Bereich von 50 bis 300 Metern pro Sekunde durch wenigstens eine Austrittsöffnung in Form von kleinen Partikeln in den Behandlungsraum ausgestoßen werden kann.

Da häufig gleichzeitig ölige und wässrige Aerosole appliziert werden sollen, weist der Aerosolerzeuger der erfindungsgemäßen Vorrichtung bevorzugt Mittel zum Zerstäuben von wenigstens zwei unterschiedlichen Fluiden auf.

Gemäß einer Variante der erfindungsgemäßen Vorrichtung umfassen die Mittel zum Zerstäuben von wenigstens zwei unterschiedlichen Fluiden eine Hochdruckpumpe, welche eine erste Hochdruckleitung mit einem ersten Fluid und eine zweite Hochdruckleitung mit einem zweiten Fluid versorgt. Vorzugsweise mündet die erste Hochdruckleitung in wenigstens einer ersten Austrittsöffnung und die zweite Hochdruckleitung in wenigstens einer zweiten Austrittsöffnung.

Gemäß einer Variante der erfindungsgemäßen Vorrichtung umfasst der Aerosolerzeuger wenigstens eine erste Hochdruckkammer, in welcher die mindestens eine Austrittsöffnung angeordnet ist, wobei die Austrittsöffnung als verschließbare Austrittsöffnung ausgebildet ist, die nach Erreichen eines ersten Innendrucks in der Hochdruckkammer öffnet, und eine zweite Hochdruckkammer, in welcher die wenigstens eine zweite Austrittsöffnung angeordnet ist, die wiederum als verschließbare Austrittsöffnung ausgebildet ist und bei Erreichen eines zweiten Innendrucks in der zweiten Hochdruckkammer öffnet. Bei dieser Ausführungsform versorgt die Hochdruckpumpe die erste Hochdruckkammer über die erste Hochdruckleitung mit dem ersten Fluid und die zweite Hochdruckkammer über die zweite Hochdruckleitung mit dem zweiten Fluid. Mit der erfindungsgemäßen Vorrichtung können daher mittels einer einzigen Hochdruckpumpe wenigstens zwei unterschiedliche Fluide, vorzugsweise genau zwei unterschiedliche Fluide, zerstäubt werden. Gegenüber der aus WO 00/44331 A1 bekannten Vorrichtung ist daher eine kostengünstigere Herstellung der Zerstäubervorrichtung möglich.

Die Hochdruckpumpe der erfindungsgemäßen Vorrichtung weist vorteilhaft einen ersten Zylinder, in welchem die erste Hochdruckleitung und eine erste Zuleitung für das erste Fluid mündet, und einen zweiten Zylinder, in welchen die zweite Hochdruckleitung und eine zweite Zuleitung für das zweite Fluid mündet, auf. Zur Kompression der in dem ersten bzw. zweiten Zylinder befindlichen Fluide umfasst die Hochdruckpumpe vorteilhaft einen ersten Kolben, der in den ersten Zylinder eingreift und dort gegen die Umgebung abgedichtet verschiebbar ist, sowie einen zweiten Kolben, der in den zweiten Zylinder eingreift und dort ebenfalls gegen die Umgebung abgedichtet verschiebbar ist. Der erste und zweite Kolben werden dabei gemeinsam von einem einzigen Motor angetrieben.

Gemäß einer bevorzugten Ausführungsform der erfindungsgemäßen Vorrichtung münden die ersten und zweiten Zuleitungen über erste bzw. zweite Einlassöffnungen in den ersten bzw. zweiten Zylinder, wobei die erste Einlassöffnung durch den ersten Kolben und die zweite Einlassöffnung durch den zweiten Kolben verschließbar ist. Damit wird die Zufuhr der zu zerstäubenden Fluide über die Kolbenbewegung gesteuert. Vorzugsweise sind die erste Einlassöffnung und die zweite Einlassöffnung in einem Abschnitt des Zylinders angeordnet, der von zwei Dichtungsringen begrenzt wird, die gemeinsam mit dem jeweiligen Kolben die eigentliche Abdichtung der Zuleitung gewährleisten.

Eine besonders einfache Ausgestaltung der erfindungsgemäßen Vorrichtung erhält man, wenn für die zu zerstäubenden Fluide Reservoire vorgesehen sind und die Einleitung der Fluide in den Zylinder allein mittels Schwerkraft erfolgt. Dazu sind bei einer bevorzugten Ausführungsform der erfindungsgemäßen Vorrichtung ein erstes Reservoir für das erste Fluid und ein zweites Reservoir für das zweite Fluid so angeordnet, dass das erste bzw. zweite Fluid bei geöffneter erster bzw. zweiter Einlassöffnung unter der Einwirkung der Schwerkraft durch die erste bzw. zweite Zuleitung aus dem ersten bzw. zweiten Reservoir in den ersten bzw. zweiten Zylinder abfließen kann. Dazu wird der erste bzw. zweite Kolben soweit aus dem Zylinder herausgezogen, dass einerseits die erste bzw. zweite Einlassöffnung freigegeben wird, aber andererseits durch den äußersten der Dichtungsringe noch eine Abdichtung des Zylinders nach außen gewährleistet ist.

Beim Herausziehen der Kolben aus dem Zylinder bis zur äußersten Abdichtung entsteht in den Zylindern ein Unterdruck, der das Einströmen der zu zerstäubenden Fluide begünstigt. Da die Dichtungen zwar flüssigkeitsdicht, aber nicht notwendigerweise luftdicht sind, sammelt sich durch unvermeidliche Leckagen auch Luft in dem Zylinder an, die entweichen muss, wenn sich der Zylinder langsam mit dem zu zerstäubenden Fluid füllt. Dazu sind in dem ersten Zylinder und/oder in dem zweiten Zylinder, vorzugsweise in beiden Zylindern, Entlüftungsöffnungen vorgesehen. Vorzugsweise sind die Entlüftungsöffnungen in dem von den beiden Dichtungen begrenzten Zylinderabschnitt angeordnet. Beim Herausziehen der Kolben bis zur äußeren Dichtung werden dann auch die Entlüftungsöffnungen freigegeben und die in den Zylindern vorhandene Luft kann beim Einströmen der zu zerstäubenden Fluide in den Zylinder über die Entlüftungsöffnungen entweichen.

Gemäß der bisher beschriebenen Varianten der erfindungsgemäßen Vorrichtung werden die beiden Fluide gleichzeitig ausgegeben, da beide Kolben von einem Linearmotor angetrieben werden. Über geeignete zwischengeschaltete Umlenkungen, Übersetzungen oder Ähnliches kann man selbstverständlich auch erreichen, dass die beiden Kolben alternierend in die Zylinder eingefahren und herausgezogen werden, d. h. dass während der Kompression des ersten Fluids im ersten Zylinder der zweite Zylinder mit dem zweiten Fluid gefüllt wird und umgekehrt. Eine besonders flexible Ausgestaltung der erfindungsgemäßen Vorrichtung erhält man aber, wenn in der ersten Zuleitung für das erste Fluid und/oder in der zweiten Zuleitung für das zweite Fluid ein steuerbares Zulaufventil angeordnet ist, so dass das Befüllen des Zylinders über das Zulaufventil unabhängig von der Kolbenbewegung gesteuert werden kann. So hat es sich beispielsweise herausgestellt, dass es bei Behandlungen mit Salzwasseraerosolen und Ölaerosolen vorteilhaft ist, wenn zunächst das Salzwasseraerosol auf die Haut einwirkt und erst anschließend das Ölaerosol, weil das Öl die Poren der Haut verschließt und die Wirkung des Salzwasseraerosols mindert. Wenn es sich daher bei dem ersten zu zerstäubenden Fluid um Salzwasser handelt und bei dem zweiten zu zerstäubenden Fluid um ein Öl, ist vorteilhaft in der zweiten Zuleitung für das Öl ein steuerbares Zulaufventil angeordnet. Die Steuerung des Zulaufventils kann so ausgelegt sein, dass das Zulaufventil für das Öl erst dann öffnet, wenn eine bestimmte, vorgegebene Anzahl von Kolbenhüben erfolgt ist. Typischerweise wird die vorgegebene Anzahl von Kolbenhüben so bemessen sein, dass gewährleistet ist, dass das Salzwasserreservoir vollständig entleert ist, bevor das Öl zerstäubt wird. Bei einer aufwändigeren Variante kann dazu das Zulaufventil für das Öl über einen Niveauwächter im Salzwasserreservoir gesteuert werden. Alternativ ist auch eine Zeitsteuerung des Zulaufventils möglich. Wenn beispielsweise in beiden Zuleitungen steuerbare Zulaufventile angeordnet sind, können die Zulaufventile so ausgelegt sein, dass eine gewisse Zeit nur das erste Ventil geöffnet ist und anschließend für eine gewisse Behandlungsdauer nur das zweite Ventil. Diese Variante ist besonders dann bevorzugt, wenn die Fluidreservoire nicht jeweils einer einzigen Behandlung entsprechen, sondern so dimensioniert sind, dass zahlreiche Behandlungen mit den in den Reservoiren befindlichen Fluidvorräten durchgeführt werden können.

Um zu gewährleisten, dass die Hochdruckkammern erst bei Erreichen eines vorgegebenen Innendrucks öffnen, sind die Austrittsöffnungen jeweils durch in Verschlussrichtung vorgespannte Dorne verschlossen. Beispielsweise können die Dorne eine konusförmige Spitze aufweisen, während die Austrittsöffnungen einen konusförmigen Sitz für die Dorne bilden. Bei Erreichen des vorgegebenen Innendrucks löst sich der Dorn vom Sitz und gibt einen ringförmigen Austrittsspalt frei, über den das in der Hochdruckkammer enthaltene Fluid zerstäubt wird. Vorzugsweise werden die Dorne jeweils durch entsprechende Spiralfedern vorgespannt, die so in der Hochdruckkammer angeordnet sind, dass ihre Vorspannung, d. h. der von den Spiralfedern auf die Dorne ausgeübte Druck, von außen einstellbar ist.

Gemäß einer besonders einfachen Ausführungsform der erfindungsgemäßen Vorrichtung sind jedoch keine verschließbaren Hochdruckkammern vorgesehen, sondern die erste und zweite Hochdruckleitung münden direkt ohne Zwischenschaltung von Hochdruckkammern in die erste bzw. zweite Austrittsöffnung der Austrittsdüsen.

Gemäß einer besonders bevorzugten Variante der erfindungsgemäßen Vorrichtung ist das zu zerstäubende Fluid in einer Kartusche angeordnet, welche die wenigstens eine Austrittsöffnung zum Zerstäuben des Fluids und einen beweglichen Kolben aufweist, der durch eine Antriebseinrichtung bewegt werden kann. Die Kartusche ist vorteilhaft auswechselbar ausgebildet, so dass die erfindungsgemäße Vorrichtung nach Gebrauch durch einfaches Auswechseln der Kartusche wieder in einen einsatzbereiten Zustand versetzt werden kann. Die Kartusche kann als Einwegkartusche oder als wieder verwendbare Kartusche ausgebildet sein. Die Kartusche kann mit einem Innendruck von mehreren hundert Bar beaufschlagt werden. Die Kartusche kann aus jeglichem inerten, druck- und chemikalienbeständigen Material bestehen, beispielsweise aus Kunststoff, Metall, Glas oder Keramik. Besonders bevorzugt ist die Kartusche ein spritzgegossenes Kunststoffteil, beispielsweise aus PEEK. Gegebenenfalls kann die Kartusche in einer die Kartusche ganz oder teilweise umschließenden Metallhalterung angeordnet werden, um die nötige Druckfestigkeit zu gewährleisten. Typischerweise fasst die Kartusche ein Volumen des zu zerstäubenden Fluids, das im Bereich von 20 bis 40 ml für Humananwendungen und bei etwa 100 ml bis zu mehreren hundert ml bei Großtieranwendungen liegen kann. Vorteilhaft wird die Kartusche schon vom Hersteller vorgefüllt, so dass die Einhaltung von hygienischen und arzneimitteltechnischen Anforderungen gewährleistet ist. Gemäß einer Variante enthält die Kartusche lediglich einen Zylinder, so dass für die Abgabe von mehreren unterschiedlichen Fluiden nacheinander oder gleichzeitig mehrere Kartuschen verwendet werden müssen. Eine Kartusche kann jedoch auch als Mehrzylinderkartusche ausgebildet sein, so dass auch mittels einer Kartusche die gleichzeitige Abgabe von mehreren unterschiedlichen Fluiden ermöglicht wird.

Als Antriebsmotor wird vorzugsweise ein Niederspannungsmotor, besonders bevorzugt ein Niederspannungs-Linearmotor, wie beispielsweise ein Niederspannungs-Elektrozylinder verwendet. Der Niederspannungs-Elektrozylinder kann beispielsweise bei einer Betriebsspannung von 24 V betrieben werden. Damit ist ein sicherer Betrieb in feuchter Umgebung, beispielsweise in einem Nebel aus Aerosolteilchen, die aus einer wässrigen Salzlösung erzeugt wurden, möglich. Ein Elektrozylinder als Antriebsmotor weist außerdem den Vorteil auf, dass ein solcher Motor völlig gekapselt ausgeführt sein kann, wobei lediglich die Antriebsspindel durch eine geeignete Dichtung abgedichtet aus dem geschlossenen Gehäuse herausgeführt ist. Es besteht daher nicht die Gefahr, dass Aerosolteilchen der wässrigen Salzlösung in den Elektrozylinder eindringen und zu Korrosion oder zu Kurzschlüssen führen.

Mit der erfindungsgemäßen Vorrichtung können unterschiedlichste Fluide zerstäubt werden. Vorzugsweise werden aber Kombinationen aus wässrigen Lösungen und öligen Lösungen zerstäubt. Besonders bevorzugt handelt es sich daher bei dem ersten Fluid um eine wässrige Lösung, beispielsweise Wasser oder eine wässrige Salzlösung. Bei dem zweiten Fluid handelt es sich vorzugsweise um ein Öl, vorzugsweise um ein Zusatzstoffe enthaltendes Öl. Typische Zusatzstoffe können beispielsweise ätherische Substanzen, Vitamine, pharmazeutisch wirksame Substanzen und Ähnliches sein. Wenn es sich um Zusatzstoffe handelt, die eher in wässriger Lösung löslich sind, kann selbstverständlich auch das erste Fluid entsprechende Zusatzstoffe enthalten. Gegenstand der Erfindung ist außerdem ein Nebelerzeuger zur Behandlung einer Person oder eines Tiers mit wässrigen und öligen Aerosolen, der dadurch gekennzeichnet ist, dass der Nebelerzeuger eine Vorrichtung zum Zerstäuben von wenigsten zwei unterschiedlichen Fluiden der oben definierten Art zur Erzeugung eines Wasser- und Ölnebels umfasst.

Der Nebelerzeuger kann in einem Raum, beispielsweise in einem Pferdestall, einer Hundehütte oder einem Behandlungsraum für Personen angeordnet werden, in welchem der Aerosolnebel erzeugt werden soll. Gemäß einer anderen Variante kann der Nebelerzeuger Teil einer Behandlungskabine sein oder selbst eine Behandlungskammer umfassen, die den ganzen Körper oder einen Teil des Körpers der zu behandelnden Person oder des zu behandelnden Tiers umschließen kann.

Zum Zerstäuben werden die Fluide vorzugsweise mit einem Druck von mindestens 100 bar, besonders bevorzugt mit einem Druck zwischen 100 und 800 bar, und ganz besonders bevorzugt mit einem Druck zwischen 400 und 600 bar, beispielsweise mit einem Druck von etwa 570 bar beaufschlagt. Beim plötzlichen Entspannen der so komprimierten Flüssigkeit auf Umgebungsdruck entstehen Aerosolteilchen, die typischerweise eine Größe zwischen 0,5 und 10 µm³ und besonders bevorzugt von einem etwa 1 µm³ haben.

Die typischen im Rahmen einer Behandlung zerstäubten Flüssigkeitsmengen hängen von den Abmessungen des Behandlungsraums, in welchem der Aerosolnebel erzeugt werden soll, ab. Für Teilkörperbehandlungen einer Person reichen Flüssigkeitsmengen von wenigen Millilitern bereits aus. Für die Behandlung einer Person in einer Aerosolkabine wie sie beispielsweise in WO 00/44331 A1 dargestellt ist, werden typischerweise etwa 25 ml Flüssigkeit zerstäubt, während bei der Behandlung größerer Tiere, wie beispielsweise von Pferden in entsprechenden Stallungen, einige hundert Milliliter Flüssigkeit pro Behandlung zerstäubt werden können.

Die Erfindung wird im Folgenden an Hand eines in der beigefügten Zeichnung dargestellten Ausführungsbeispiels näher erläutert. In der Zeichnung zeigt
- Figur 1: eine bevorzugte Ausführungsform der erfindungsgemäßen Vorrichtung zum Zerstäuben von zwei unterschiedlichen Fluiden im Teilausriss;
- Figur 2: eine Variante der Vorrichtung der Figur in einer Teilansicht;
- Figur 3: eine Variante der erfindungsgemäßen Vorrichtung mit kartuschenartig ausgebildeten Zerstäubungsmitteln;
- Figur 4: eine Variante der Vorrichtung der Figur 3; und
- Figur 5: eine Behandlungskammer, der zur Durchführung des erfindungsgemäßen Verfahrens mit der erfindungsgemäßen Vorrichtung geeignet ist.

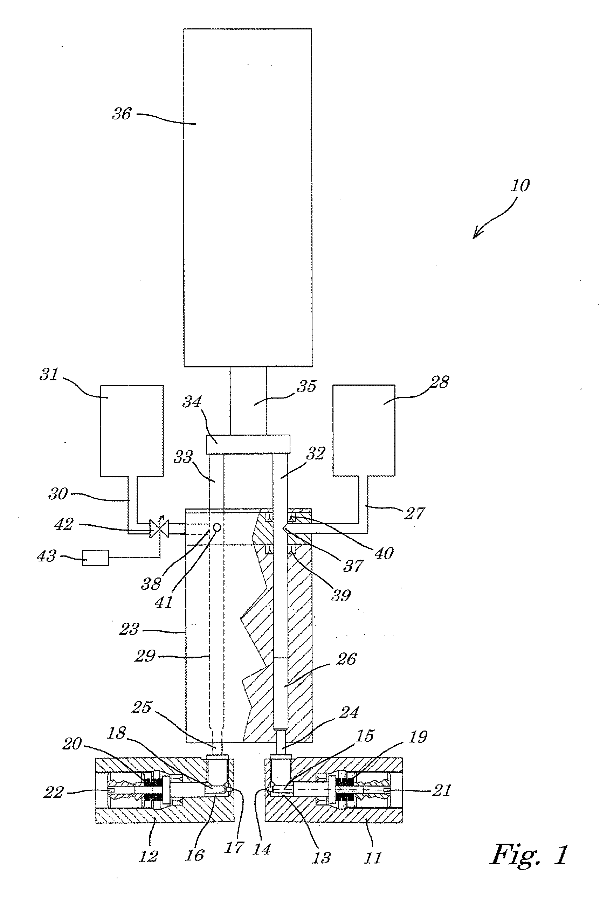

In Figur 1 ist die erfindungsgemäße Vorrichtung zum Zerstäuben von zwei unterschiedliche Fluiden insgesamt mit der Bezugsziffer 10 bezeichnet. Die Vorrichtung 10 weist einen ersten Zerstäuberkopf 11 und einen zweiten Zerstäuberkopf 12 auf. In dem ersten Zerstäuberkopf 11 ist eine erste Hochdruckkammer 13 ausgespart, die eine Austrittsöffnung 14 aufweist, die durch einen beweglichen Dorn 15 verschlossen ist. Ebenso weist der zweite Zerstäuberkopf 12 eine zweite Hochdruckkammer 16 auf, die eine Austrittsöffnung 17 zur Umgebung besitzt, welche ebenfalls durch einen beweglichen Dorn 18 verschlossen ist. Der Dorn 15 wird mittels einer Spiralfeder 19 gegen die Austrittsöffnung 14 vorgespannt, während der Dorn 18 über eine Spiralfeder 20 gegen die Austrittsöffnung 17 vorgespannt wird. Die Vorspannungen der Spiralfedern 19 bzw. 20 sind über Schraubgewinde 21 bzw. 22 von außen einstellbar. Wie in der Querschnittsdarstellung der Figur 1 erkennbar, bilden die Austrittsöffnungen 14 bzw. 17 konusförmige Sitze für die konusförmigen Spitzen der Dorne 15 bzw. 18. Der Durchmesser der Austrittsöffnungen 14,17 beträgt im dargestellten Beispiel etwa 0,5 mm. Die Spiralfedern 19 bzw. 20 sind im Beispiel so vorgespannt, dass dann, wenn der Innendruck in den Hochdruckkammern 13 bzw. 16 570 bar übersteigt, die Dorne gegen die Vorspannung der Federn von ihren Sitzen weggedrückt werden und ringförmige Austrittsschlitze am Rand der Austrittsöffnungen 14,17 freigeben, durch die die in den Hochdruckkammern 13 bzw. 16 befindlichen Fluide explosionsartig in die Umgebung freigesetzt und auf Grund der auf Umgebungsdruck abfallenden Druckverringerung in feine Tröpfchen zerstäubt werden.

Zur Erzeugung des Drucks von etwa 570 bar in den Hochdruckkammern 13 bzw. 16 weist die erfindungsgemäße Vorrichtung eine Hochdruckpumpe 23 auf, welche die erste Hochdruckkammer 13 über eine Hochdruckleitung 24 mit dem ersten Fluid und die zweite Hochdruckpumpe 16 über eine Hochdruckleitung 25 mit dem zweiten Fluid versorgt.

In der Hochdruckpumpe 23 befindet sich ein erster Zylinder 26, in dessen stirnseitiges Ende die Hochdruckleitung 24 mündet und in dessen gegenüberliegenden Endbereich eine erste Zuleitung 27 mündet, die von einem Reservoir 28 ausgeht, in welchem das erste Fluid angeordnet ist. Entsprechend ist in der Hochdruckpumpe 23 ein in dem Teilausriss der Figur 1 nur gestrichelt dargestellter zweiter Zylinder 29 vorgesehen, in den einerseits die zweite Hochdruckleitung 25 und andererseits eine zweite Zuleitung 30 mündet, die den zweiten Zylinder 29 mit einem Reservoir 31 für das zweite Fluid verbindet.

Im dargestellten Beispiel befindet sich in dem ersten Reservoir 28 eine wässrige Salzlösung und in dem zweiten Reservoir 31 Olivenöl.

In den ersten Zylinder 26 greift ein erster Kolben 32 ein, während in den zweiten Zylinder 29 ein zweiter Kolben 33 eingreift. Die beiden Kolben 32,33 sind über eine Querstrebe 34 mit der linear beweglichen Spindel 35 eines bei 24 V betriebenen Niederspannungs-Elektrozylinders 36 verbunden. Der Niederspannungs-Elektrozylinder bewirkt die Hin- und Herbewegung der Kolben 32,33 in den Zylindern 26,29 der Hochdruckpumpe 23.

Die erste Zuleitung 27 mündet über eine erste Einlassöffnung 37 in den seitlichen Endbereich des ersten Zylinders 26. Entsprechend mündet die zweite Zuleitung 30 über eine zweite Einlassöffnung 38 in den Endbereich des zweiten Zylinders 29. Der Einmündungsabschnitt der Zuleitung 27 ist in Richtung zum Zerstäuberkopf 11 mittels eines ersten Dichtungsrings 39 und zum Elektrozylinder 36 hin über einen zweiten Dichtungsring 40 abgedichtet. Der Kolben 32 wird in den Dichtungsringen 39 und 40 flüssigkeitsdicht geführt. Entsprechende Dichtungsringe sind im Bereich der zweiten Einlassöffnung 38 angeordnet, aber in Figur 1 auf Grund der Teilausrissdarstellung nicht erkennbar. Zwischen den jeweiligen Dichtungsringpaaren sind außerdem Entlüftungsöffnungen 41 angeordnet, durch welche die Zylinder 26,29 mit der Umgebung kommunizieren können (in Figur 1 ist nur die Entlüftungsöffnung 41 des zweiten Zylinders 29 dargestellt, während eine entsprechende, im Teilausriss der Figur 1 nicht dargestellte Entlüftungsöffnung auch für den ersten Zylinder 26 vorgesehen ist.

In der zweiten Zuleitung 30 ist ein steuerbares Zulaufventil 42 angeordnet, das über eine Kontrolleinheit 43 programmierbar ist.

Im Folgenden wird die Funktionsweise der in Figur 1 dargestellten Ausführungsform zum Zerstäuben von einer in dem Reservoir 28 befindlichen wässrigen Salzlösung und dem im Reservoir 31 befindlichen Olivenöl beschrieben:

Die beiden Reservoire 28,31 sind dabei so dimensioniert, dass die Flüssigkeiten für eine einzelne Behandlung darin vorgehalten werden können.

Die entsprechenden Flüssigkeiten können aus vorkonfektionierten Phiolen in die Reservoire 28,31 geführt werden. Alternativ können die Leitungen 27,30 in (in Figur 1 nicht dargestellten) Anschlussstutzen enden, an welche vorkonfektionierte Flüssigkeitsbehälter angeschlossen werden können. Die Vorrichtung 10 und die Flüssigkeitsreservoire 28 und 38 sind im Beispiel der Figur 1 so angeordnet, dass die im Reservoir 28 befindliche Salzlösung und das im Reservoir 31 befindliche Öl unter der Einwirkung der Schwerkraft in die senkrecht orientierten Zylinder 26 bzw. 29 abfließen können. In dem in Figur 1 dargestellten Zustand wird das Abfließen der Flüssigkeiten aus den Reservoiren jedoch durch die Kolben 32 bzw. 33 verhindert, welche die Entlüftungsöffnungen 37 bzw. 38 blockieren. Nach Einschalten der Vorrichtung 10 versetzt der Elektrozylinder 36 über seine Spindel 35 die damit verbundenen Kolben 32 und 33 in eine reziproke Bewegung. In einer ersten Betriebsphase wird das Zulaufventil 42 geschlossen gehalten, so dass unabhängig von der Stellung des Kolbens 33 kein Öl in den Zylinder 29 gelangen kann. Sobald in dieser ersten Betriebsphase der Kolben 32 so weit aus dem Zylinder 26 herausgezogen wird, dass die Einlassöffnung 37 freigegeben wird, fließt auf Grund des beim Herausziehen des Kolbens 32 in dem Zylinder 26 entstandenen Unterdrucks und der Einwirkung der Schwerkraft wässrige Salzlösung aus dem Behälter 28 in den Zylinder 26. Gleichzeitig kann in dem Zylinder 26 enthaltene Luft über die (nicht dargestellte) Entlüftungsöffnung des Zylinders 26 entweichen. Der Kolben 32 wird dabei maximal bis zu dem zweiten Dichtungsring 40 herausgezogen, so dass stets eine Abdichtung zur Umgebung gewährleistet ist. Die in den Zylinder 26 einströmende Salzlösung gelangt dann über die Hochdruckleitung 34 auch in die erste Hochdruckkammer 13. Solange lediglich Umgebungsdruck anliegt, kann die Salzlösung die Hochdruckkammer 13 nicht verlassen. Sobald der Kolben 32 wieder in den Zylinder 26 hinein gefahren wird, entsteht in dem Zylinder 26, der Hochdruckleitung 24 und der Hochdruckkammer 13 ein Druck, der so lange ansteigt, bis der die Austrittsöffnung 14 verschließende Dorn 15 gegen die Vorspannung der Spiralfeder 19 von seinem Sitz abgehoben und ein ringförmiger Austrittspalt an der Austrittsöffnung 14 freigegeben wird. Im dargestellten Beispiel ist die Feder 19 so vorgespannt, dass die Austrittsöffnung 14 bei einem Druck von 570 bar freigegeben wird. Nach Ausstoßen der Flüssigkeit sinkt der Druck wieder unter die vorgegebene Grenze und der Dorn 15 verschließt die Austrittsöffnung wieder dicht. Anschließend wird der Kolben 32 wieder bis zum zweiten Dichtungsring 40 herausgezogen und weitere Salzlösung kann aus dem Reservoir 28 nachfließen. Dieser Vorgang wird so lange wiederholt, bis gewährleistet ist, dass das im Reservoir 28 ursprünglich enthaltene Volumen an wässriger Salzlösung zerstäubt worden ist. Die Anzahl der Hubvorgänge kann dabei aus den Volumen der ursprünglich vorhandenen Salzlösungen und dem pro Kolbenhub zerstäubten Volumen errechnet werden. Sobald die Steuerung 43 des Zulaufventils 42 die entsprechende Anzahl der Hubvorgänge registriert hat (beispielsweise mittels (nicht dargestellter) Kontaktschalter), öffnet die Steuerung 43 das Zulaufventil 42, so dass beim nächsten Herausziehen des Kolbens 33 aus dem zweiten Zylinder 29 Öl aus dem Reservoir 31 in den zweiten Zylinder 29, die zweite Hochdruckleitung 25 und die zweite Hochdruckkammer 16 fließen kann. Wie im Fall der Salzlösung in der ersten Hochdruckkammer 13 wird nun durch Komprimieren des Öls und einer entsprechenden Einstellung der zweiten Spiralfeder gewährleistet, dass das Öl bei einem Druck von etwa 570 bar über die zweite Austrittsöffnung 17 in Form eines fein verteilten Aerosolnebels in den Behandlungsraum abgegeben wird. Auch dieser Vorgang wird so lange wiederholt, bis die gewünschte Menge an Öl zerstäubt worden ist.

Das beschriebene Verfahren gewährleistet, dass zwar einerseits Salzlösung und andererseits Öl mit ein und derselben Vorrichtung zerstäubt werden können, dass aber nur Salzlösung und anschließend nur Öl abgegeben werden, was eine optimale therapeutische Wirkung gewährleistet.

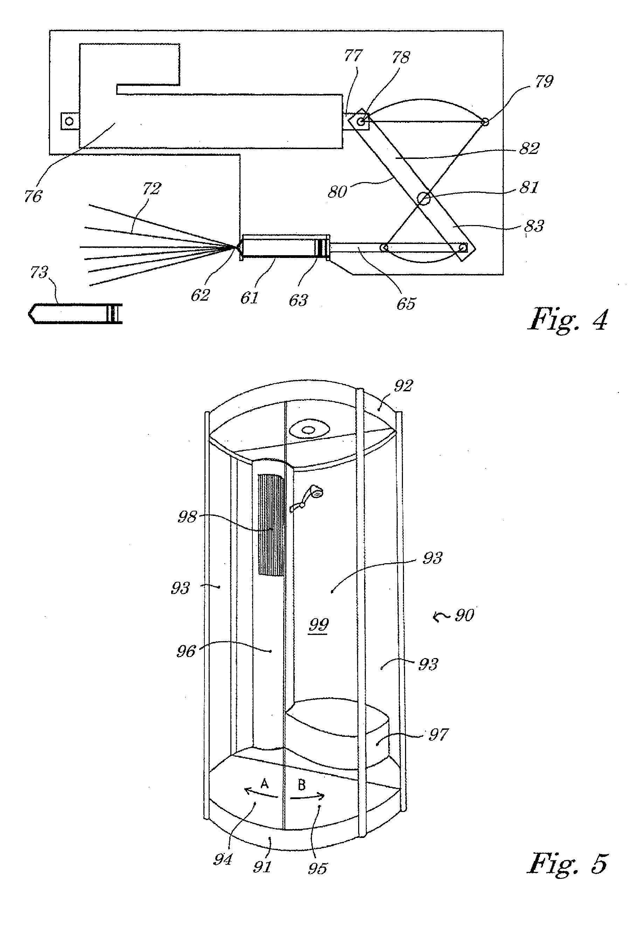

Es versteht sich, dass der an den Austrittsöffnungen 14 bzw. 17 erzeugte Aerosolnebel nicht unmittelbar in den Behandlungsraum abgegeben werden muss, sondern das beispielsweise eine Vorkammer vorgesehen sein kann, aus welcher der Aerosolnebel über einen zusätzlichen Ventilator in den eigentlichen Behandlungsraum abgegeben wird, wie dies beispielsweise in der WO 00144331 A1 dargestellt ist. Ebenso können die dort ebenfalls beschriebenen Schalldämpfer zur Geräuschminimierung vorgesehen sein. Eine typische Aerosolbehandlungskabine, in welcher die erfindungsgemäße Zerstäubungsvorrichtung angeordnet sein kann, wird weiter unten im Zusammenhang mit der Figur 5 beschrieben und ist ebenfalls in WO 00/44331 A1 ausführlicher dargestellt.

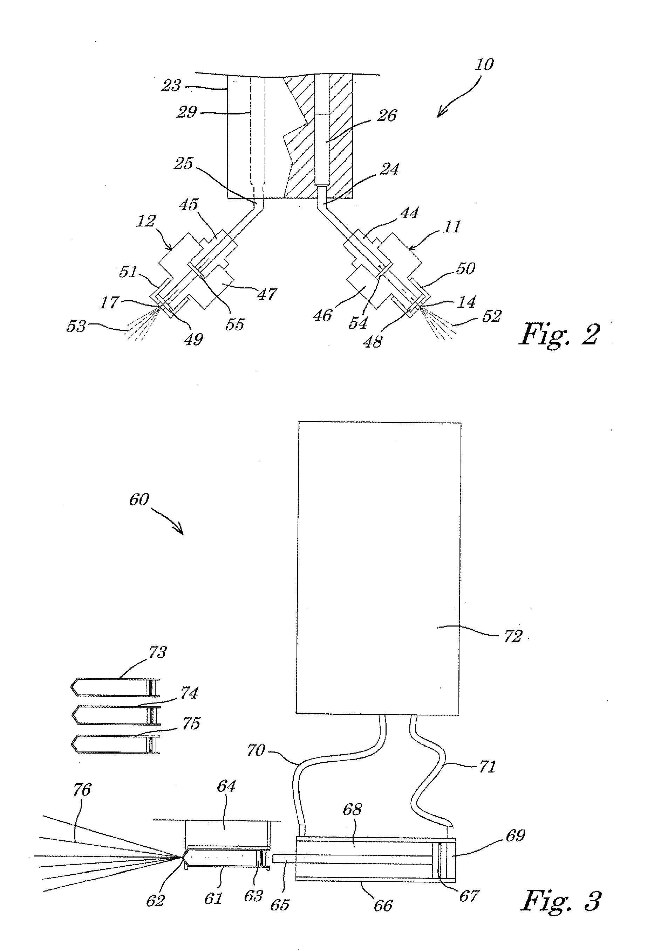

In Figur 2 ist eine Variante der Vorrichtung der Figur 1 in einer Teilansicht dargestellt, wobei Bauteile, welche die gleiche oder eine entsprechende Funktion wie Bauteile der Vorrichtung der Figur 1 erfüllen, mit denselben Bezugsziffern bezeichnet sind. Die Variante der Figur 2 unterscheidet sich von Figur 1 lediglich in der Gestaltung der Zerstäuberköpfe 11, 12, die im vorliegenden Fall besonders einfach ausgebildet sind, da die Hochdruckleitungen 24, 25 über ein Anschlussstück 44, 45 direkt in den Düsenstock 46, 47 münden, wo jeweils eine Metallplatte 48, 49 mit den Düsenöffnungen 14, 17 angeordnet ist. Die Metallplatte ist jeweils durch eine Überwurfmutter 50, 51 gesichert. Mit den Bezugsziffern 52, 53 sind in Figur 2 die aus den Düsen 14, 17 austretenden Aerosolpartikel dargestellt. In den Hochdruckleitungen 24, 25 können, wie beim dargestellten Beispiel, Filter 54, 55 angeordnet werden, welche größere Partikel aus den zu zerstäubenden Fluiden herausfiltern, so dass ein Verstopfen oder gar eine Beschädigung der Düsen verhindert wird. Bei der in Figur 2 dargestellten Ausführungsform sind jeweils ein Filter 54, 55 im Bereich des Übergangs der Hochdruckleitungen 54, 55 von dem Anschlussstück 44, 45 in den Düsenstock 46, 47 angeordent.

Figur 3 zeigt eine Variante der erfindungsgemäßen Vorrichtung zum Zerstäuben von Fluiden, die insgesamt mit der Bezugsziffer 60 bezeichnet ist. Bei der Variante der Zerstäubungsvorrichtung 60 befindet sich das zu zerstäubende Fluid in einer Kartusche 61, die eine Austrittsdüse 62 und einen am gegenüberliegenden Ende der Kartusche angeordneten beweglichen Stempel 63 aufweist. Die Kartusche 61 ist auswechselbar in einer Halterung 64 angeordnet, in welcher sie so positioniert ist, dass der endseitige Stempel 63 der Kartusche von einem Schaft 65 eines Hydraulikzylinders 66 beaufschlagt werden kann. Der Schaft 65 ist mit einem beweglichen Kolben 67 verbunden, der in dem Hydraulikzylinder 66 hin- und herbewegt werden kann. Der Kolben 67 teilt den Hydraulikzylinder in zwei Kammern 68, 69, welche über Hydraulikleitungen 70, 71 mittels eines Hydraulikantriebs 72 mit einer Hydraulikflüssigkeit beaufschlagt werden können, um den Kolben 67 in an sich bekannter Weise hin- und herzubewegen. Mit den Bezugsziffern 73, 74 und 75 sind in Figur 3 weitere Ersatzkartuschen bezeichnet, welche nach Gebrauch der Kartusche 61 in die Halterung 64 eingesetzt werden können. Mit 76 ist in Figur 3 das durch die Austrittsöffnung 62 der Kartusche 61 zerstäubte Fluid symbolisiert.

Figur 4 zeigt eine Variante der Vorrichtung der Figur 3, die sich lediglich durch den Antrieb des Schachtes 65 von der in Figur 3 beschriebenen Ausführungsform unterscheidet. Bauelemente, welche gleiche oder ähnliche Funktionen wie Bauelemente der Ausführungsform der Figur 3 erfüllen, sind mit denselben Bezugsziffern bezeichnet und werden nicht nochmals beschrieben. Anstelle des bei der Ausführungsform der Figur 3 vorgesehenen Hydraulikantriebs wird der Kolbenschaft 65 der Variante der Figur 4 rein mechanisch betrieben. Die Antriebseinrichtung weist dazu einen Linearmotor 76 auf, der einen Schaft 77 zwischen einer Ausgangsposition 78 und einer Endposition 79 geradlinig hin- und herbewegen kann. An der Spitze des Schaftes 77 ist ein Querträger 80 angelenkt, dessen gegenüberliegendes Ende am Ende des Schaftes 65 angelenkt ist. Der Querträger 80 ist an einem Drehpunkt 81 drehbar fixiert, so daß zwei Hebelarme 82, 83 gebildet werden, deren Verhältnis die von dem Schaft 77 auf den Schaft 65 übertragene Kraft bestimmt.

Figur 5 zeigt schließlich eine Behandlungskammer 90, in welche eine oder mehrere der erfindungsgemäßen Vorrichtungen zur Zerstäubung eines therapeutisch wirksamen Aerosols eingebaut sein können. Die in Fig. 5 dargestellte Behandlungskammer 90 ist detaillierter in der internationalen Patentanmeldung WO 00/44331 A1 beschrieben.

Die Behandlungskammer 90 wird von einer Bodenplatte 91, einer Deckenplatte 92, Seitenscheiben 93 aus Acrylglas und zwei entlang der Pfeile A, B beweglichen Vorderscheiben 94, 95, die den Zugang zu der Behandlungskammer 90 ermöglichen, begrenzt.

In der Behandlungskammer 90 erstreckt sich zwischen der Bodenplatte 91 und der Deckenplatte 92 ein Rohr 96. Zudem ist an einer Oberseite der Bodenplatte 91 eine wasserdichte Sitzbank 97 angeordnet. In dem Rohr 96 und in der Sitzbank 97 sind die (in Figur nicht erkennbaren) Vorrichtungen zur Aerosolerzeugung und -verteilung einschließlich entsprechender, in WO 00/44331 A1 näher beschriebener Hilfseinrichtungen, wie Schalldämpfer und Ventilatoren 35 angeordnet.

Mit einer Vorrichtung, wie sie im Zusammenhang mit den Figuren 1 bis 4 beschrieben wurde, wird das zu zerstäubende Fluid in Form von Aerosolpartikeln ausgestoßen. Die Aerosolpartikel strömen dann zu einem oberen Ende des Rohrs 96, und von dort über ein Gitter 98 in den Innenraum 99 der Behandlungskammer 90. Beispielsweise kann durch einen (nicht dargestellten) Ventilator, der unterhalb des Gitters 98 am oberen Ende des Rohrs 96 angeordnet ist, ein nach oben gerichteter Sog erzeugt werden. Im gleichen Maß, in dem der Nebel über das Gitter 98 aus dem Rohr 96 in den Baderaum 99 strömt, kann Luft über am unteren Ende des Rohrs 96, unterhalb des oder der dort angeordneten Aerosolerzeuger, vorgesehene (nicht dargestellte) Zuluftöffnungen nachströmen.

Im Weiteren werden weitere Aspekte gemäß Ausführungsbeispielen der Erfindung offenbart:
1. Aspekt: Verfahren zum Zerstäuben wenigstens eines Fluids, insbesondere zur Erzeugung eines therapeutisch wirksamen Aerosols in einem Behandlungsraum, wobei man ein zu zerstäubendes Fluid mit Druck beaufschlagt und mit einer Fließgeschwindigkeit im Bereich von 50 bis 300 m/s durch eine Düse in Form von kleinen Partikeln in den Behandlungsraum ausstößt.
2. Aspekt: Verfahren gemäß Aspekt 1, dadurch gekennzeichnet, dass Fließgeschwindigkeit des Fluids im Bereich von 100 bis 250 m/s und bevorzugt bei etwa 150 m/s liegt.
   Verfahren gemäß einem der Aspekte 1 oder 2, dadurch gekennzeichnet, dass man das zu zerstäubende Fluid mit einem Druck beaufschlag, der bis zu 1000 bar beträgt.
3. Aspekt: Verfahren gemäß einem der Aspekte 1 bis 3, dadurch gekennzeichnet, dass die Düse wenigstens eine Austrittsöffnung mit einem Durchmesser aufweist, der im Bereich von 0,05 bis 0,2 mm, vorzugsweise im Bereich von 0,08 bis 0,15 mm und ganz besonders bevorzugt im Bereich von etwa 0,1 mm liegt.
4. Aspekt: Verfahren gemäß einem der Aspekte 1 bis 4, dadurch gekennzeichnet, dass man die ausgestoßenen Partikel einen mittleren Durchmesser aufweisen, der im Bereich von 0,05 bis 5 µm, bevorzugt im Bereich von 0,1 bis 0,9 µm und besonders bevorzugt im Bereich von 0,1 bis 0,5 µm liegt.
5. Aspekt: Verfahren gemäß einem der Aspekte 1 bis 5, dadurch gekennzeichnet, dass das Fluid eine wässrige Lösung, eine wässrige Emulsion, insbesondere eine Öl-in-Wasser-Emulsion, eine ölige Lösung, eine ölige Emulsion, insbesondere eine Wasser-in-ÖI-Emulsion, eine wässrige Suspension oder eine ölige Suspension ist.
6. Aspekt: Verfahren gemäß Aspekt 6, dadurch gekennzeichnet, dass die die wässrige Lösung eine Salzlösung umfasst.
7. Aspekt: Verfahren gemäß Aspekt 6, dadurch gekennzeichnet, dass die die ölige Lösung ätherische Öle umfasst.
8. Aspekt:Verfahren gemäß Aspekt 8, dadurch gekennzeichnet, dass das Fluid wenigstens einen therapeutisch wirksame Bestandteil umfasst.
9. Aspekt: Verfahren gemäß einem der Aspekte 1 bis 10, dadurch gekennzeichnet, dass der Behandlungsraum eine Inhalationsmaske oder eine Behandlungskammer ist, welche ein mit dem Aerosol zu behandelndes Lebewesen ganz oder teilweise umgibt.
10. Aspekt: Verfahren gemäß Aspekt 10, dadurch gekennzeichnet, dass die Behandlungskammer auf eine Temperatur im Bereich von 0 und 70°C, bevorzugt im Bereich von 20 und 35 °C temperierbar.
11. Aspekt: Verfahren gemäß einem der Aspekte 10 oder 11 , dadurch gekennzeichnet, dass das Aerosol dem zu behandelnden Lebewesen während einer Behandlungsdauer von 10 bis 30 Minuten verabreicht wird.
12. Aspekt: Verfahren gemäß einem der Aspekte 1 bis 12, wobei das Aerosol mit einer durchschnittlichen Durchflussrate im Bereich von 10 bis 100 ml/min ausgestoßen werden.
13. Aspekt: Vorrichtung zum Zerstäuben wenigstens eines Fluids, insbesondere zur Erzeugung eines therapeutisch wirksamen Aerosols mit
   wenigstens einem Aerosolerzeuger (10, 60) zur Abgabe eines Aerosols, dadurch gekennzeichnet, dass der Aerosolerzeuger (10, 60) ein zu zerstäubendes Fluid mit Druck beaufschlagt und mit einer Fließgeschwindigkeit im Bereich von 50 bis 300 m/s durch wenigstens eine Austrittsöffnung (14, 17) in Form von kleinen Partikeln ausstößt.
14. Aspekt: Vorrichtung gemäß Aspekt 13, dadurch gekennzeichnet, dass der Aerosolerzeuger (10, 60) Mittel zum Zerstäuben von wenigstens zwei unterschiedlichen Fluiden aufweist.
15. Aspekt: Vorrichtung gemäß Aspekt 14, dadurch gekennzeichnet, dass die Mittel zum Zerstäuben von wenigstens zwei unterschiedlichen Fluiden eine Hockdruckpumpe (23) umfassen, welche eine erste Hochdruckleitung (24) mit einem ersten Fluid und eine zweite Hochdruckleitung (25) mit einem zweiten Fluid versorgt.
16. Aspekt: Vorrichtung gemäß Aspekt 15, dadurch gekennzeichnet, dass die erste Hochdruckleitung (24) in wenigstens einer ersten Austrittsöffnung (14) und die zweite Hochdruckleitung in wenigstens einer zweiten Austrittsöffnung (17) mündet.
17. Aspekt: Vorrichtung gemäß Aspekt 16, dadurch gekennzeichnet, dass der Aerosolerzeuger (10)
   wenigstens eine erste Hockdruckkammer (13), in welcher die mindestens eine Austrittsöffnung angeordnet ist, die als verschließbare Austrittsöffnung (14) ausgebildet ist und bei einem ersten Innendruck öffnet,
   wenigstens eine zweite Hockdruckkammer (16), in welcher die wenigsten eine zweite Austrittöffnung angeordnet ist, die als verschließbare Austrittsöffnung (17) ausgebildet ist und bei einem zweiten Innendruck öffnet,
   umfasst, wobei die Hockdruckpumpe (23) die erste Hockdruckkammer (13) über die erste Hochdruckleitung (24) mit dem ersten Fluid und die zweite Hochdruckkammer (16) über die zweite Hochdruckleitung (25) mit dem zweiten Fluid versorgt.
18. Aspekt: Vorrichtung gemäß einem der Aspekte 13 oder 14, dadurch gekennzeichnet, dass das zu zerstäubende Fluid in einer Kartusche (61,73,74,75) angeordnet ist, welche die wenigstens eine Austrittsöffnung (62) und eine beweglichen Kolben (63) aufweist, der durch eine Antriebseinrichtung (65,72,76) bewegt werden kann.
19. Aspekt: Vorrichtung gemäß Aspekt 18, dadurch gekennzeichnet, dass die Kartusche (61 ,73,74,75) auswechselbar ist.
20. Aspekt: Vorrichtung gemäß einem der Aspekte 18 oder 19, dadurch gekennzeichnet, dass die Kartusche (61 ,73,74,75) als Einwegkartusche oder als wiederverwendbare Kartusche ausgebildet ist.
21. Aspekt: Vorrichtung gemäß einem der Aspekte 13 bis 20, dadurch gekennzeichnet, dass die Austrittsöffnung einen Durchmesser aufweist, der im Bereich von 0,05 bis 0,2 mm, vorzugsweise im Bereich von 0,08 bis 0,15 mm und ganz besonders bevorzugt im Bereich von etwa 0,1 mm liegt.
22. Aspekt: Nebelerzeuger zur Behandlung einer Person oder eines Tiers mit wässrigen und öligen Aerosolen, dadurch gekennzeichnet, dass der Nebelerzeuger eine Vorrichtung nach einem der Aspekte 13 bis 21 zur Erzeugung eines Wasser und Ölnebels umfasst.
23. Aspekt: Nebelerzeuger gemäß Aspekt 23, dadurch gekennzeichnet, dass wenigstens eine Behandlungsraum (99) vorgesehen ist, die den ganzen Körper oder einen Teil des Körpers der zu behandelnden Person oder des zu behandelnden Tiers umschließen kann, wobei das Aerosol in den Behandlungsraum abgegeben wird.

## Patentansprüche

1. Vorrichtung zum Zerstäuben wenigstens eines Fluids, insbesondere zur Erzeugung eines therapeutisch wirksamen Aerosols mit
wenigstens einem Aerosolerzeuger (10, 60) zur Abgabe eines Aerosols, **dadurch gekennzeichnet, dass** der Aerosolerzeuger (10, 60) ein zu zerstäubendes Fluid mit Druck beaufschlagt und mit einer Fließgeschwindigkeit im Bereich von 50 bis 300 m/s durch wenigstens eine Austrittsöffnung (14, 17) in Form von kleinen Partikeln ausstößt.

2. Vorrichtung gemäß Anspruch 1, **dadurch gekennzeichnet, dass** der Aerosolerzeuger (10, 60) Mittel zum Zerstäuben von wenigstens zwei unterschiedlichen Fluiden aufweist.

3. Vorrichtung gemäß Anspruch 2, **dadurch gekennzeichnet, dass** die Mittel zum Zerstäuben von wenigstens zwei unterschiedlichen Fluiden eine Hockdruckpumpe (23) umfassen, welche eine erste Hochdruckleitung (24) mit einem ersten Fluid und eine zweite Hochdruckleitung (25) mit einem zweiten Fluid versorgt.

4. Vorrichtung gemäß Anspruch 3, **dadurch gekennzeichnet, dass** die erste Hochdruckleitung (24) in wenigstens einer ersten Austrittsöffnung (14) und die zweite Hochdruckleitung in wenigstens einer zweiten Austrittsöffnung (17) mündet.

5. Vorrichtung gemäß Anspruch 4, **dadurch gekennzeichnet, dass** der Aerosolerzeuger (10)
wenigstens eine erste Hockdruckkammer (13), in welcher die mindestens eine Austrittsöffnung angeordnet ist, die als verschließbare Austrittsöffnung (14) ausgebildet ist und bei einem ersten Innendruck öffnet,
wenigstens eine zweite Hockdruckkammer (16), in welcher die wenigstens eine zweite Austrittsöffnung angeordnet ist, die als verschließbare Austrittsöffnung (17) ausgebildet ist und bei einem zweiten Innendruck öffnet,
umfasst, wobei die Hockdruckpumpe (23) die erste Hockdruckkammer (13) über die erste Hochdruckleitung (24) mit dem ersten Fluid und die zweite Hochdruckkammer (16) über die zweite Hochdruckleitung (25) mit dem zweiten Fluid versorgt.

6. Vorrichtung gemäß einem der Ansprüche 1 bis 5, **dadurch gekennzeichnet, dass** die Austrittsöffnung einen Durchmesser aufweist, der im Bereich von 0,05 bis 0,2 mm, vorzugsweise im Bereich von 0,08 bis 0,15 mm und ganz besonders bevorzugt im Bereich von etwa 0,1 mm liegt.

7. Vorrichtung gemäß einem der Ansprüche 1 bis 6, ferner aufweisend zumindest eines der folgenden Merkmale:
die Fließgeschwindigkeit des Fluids liegt im Bereich von 100 bis 250 m/s und bevorzugt bei etwa 150 m/s;
das zu zerstäubende Fluid wird mit einem Druck beaufschlagt, der bis zu 1000 bar beträgt;
die ausgestoßenen Partikel weisen einen mittleren Durchmesser auf, der im Bereich von 0,05 bis 5 µm, bevorzugt im Bereich von 0,1 bis 0,9 µm und besonders bevorzugt im Bereich von 0,1 bis 0,5 µm liegt;
das Fluid umfasst wenigstens einen therapeutisch wirksamen Bestandteil;
das Aerosol wird mit einer durchschnittlichen Durchflussrate im Bereich von 10 bis 100 ml/min ausgestoßen.

8. Vorrichtung gemäß einem der Ansprüche 1 bis 7, **dadurch gekennzeichnet, dass** das Fluid eine wässrige Lösung, eine wässrige Emulsion, insbesondere eine Öl-in-Wasser-Emulsion, eine ölige Lösung, eine ölige Emulsion, insbesondere eine Wasser-in-ÖI-Emulsion, eine wässrige Suspension oder eine ölige Suspension ist.

9. Vorrichtung gemäß Anspruch 8, ferner aufweisend zumindest eines der folgenden Merkmale:
die wässrige Lösung umfasst eine Salzlösung;
die ölige Lösung umfasst ätherische Öle.

10. Vorrichtung gemäß einem der Ansprüche 1 bis 9, **dadurch gekennzeichnet, dass** der Behandlungsraum eine Inhalationsmaske oder eine Behandlungskammer ist, welche ein mit dem Aerosol zu behandelndes Lebewesen ganz oder teilweise umgibt.

11. Vorrichtung gemäß Anspruch 10, ferner aufweisend zumindest eines der folgenden Merkmale:
die Behandlungskammer ist auf eine Temperatur im Bereich von 0 und 70 °C, bevorzugt im Bereich von 20 und 35°C temperierbar;
das Aerosol wird dem zu behandelnden Lebewesen während einer Behandlungsdauer von 10 bis 30 Minuten verabreicht.

12. Vorrichtung gemäß einem der Ansprüche 3 bis 11, **dadurch gekennzeichnet, dass** die Hochdruckpumpe einen ersten Zylinder, in welchem die erste Hochdruckleitung und eine erste Zuleitung für das erste Fluid mündet, und einen zweiten Zylinder, in welchen die zweite Hochdruckleitung und eine zweite Zuleitung für das zweite Fluid mündet, aufweist.

13. Vorrichtung gemäß Anspruch 12, **dadurch gekennzeichnet, dass** zur Kompression der in dem ersten Zylinder bzw. in dem zweiten Zylinder befindlichen Fluide die Hochdruckpumpe einen ersten Kolben, der in den ersten Zylinder eingreift und dort gegen die Umgebung abgedichtet verschiebbar ist, sowie einen zweiten Kolben umfasst, der in den zweiten Zylinder eingreift und dort ebenfalls gegen die Umgebung abgedichtet verschiebbar ist.

14. Nebelerzeuger zur Behandlung einer Person oder eines Tiers mit wässrigen und öligen Aerosolen, **dadurch gekennzeichnet, dass** der Nebelerzeuger eine Vorrichtung nach einem der Patentansprüche 1 bis 13 zur Erzeugung eines Wasser- und Ölnebels umfasst.

15. Nebelerzeuger gemäß Anspruch 14, **dadurch gekennzeichnet, dass** wenigstens ein Behandlungsraum (99) vorgesehen ist, der den ganzen Körper oder einen Teil des Körpers der zu behandelnden Person oder des zu behandelnden Tiers umschließen kann, wobei das Aerosol in den Behandlungsraum abgegeben wird.
